(19) Europäisches Patentamt
European Patent Office
Office européen des brevets

(11) Publication number: **0 049 029**
**B1**

(12) **EUROPEAN PATENT SPECIFICATION**

(45) Date of publication of patent specification: **04.09.85**

(51) Int. Cl.$^4$: **C 07 D 237/28,**
C 07 D 403/12, A 01 N 43/58

(21) Application number: **81302940.2**

(22) Date of filing: **29.06.81**

(54) **Cinnolinyloxy(amino)phenoxyalkane carboxylic acid derivatives, process for their synthesis, herbicidal compositions containing them and their use as herbicides.**

(30) Priority: **01.07.80 AU 4319/80**
**27.03.81 AU 8175/81**

(43) Date of publication of application:
**07.04.82 Bulletin 82/14**

(45) Publication of the grant of the patent:
**04.09.85 Bulletin 85/36**

(84) Designated Contracting States:
**AT BE CH DE FR GB IT LI LU NL SE**

(58) References cited:
**EP-A-0 023 785**
**EP-A-0 026 622**
**DE-A-2 745 869**
**FR-A-2 389 610**
**GB-A-1 548 847**
**GB-A-2 042 539**

(73) Proprietor: **ICI AUSTRALIA LIMITED**
**ICI House 1 Nicholson Street P.O.Box 4311**
**Melbourne Victoria 3001 (AU)**

(72) Inventor: **Serban, Alexander**
**3 Maple Court**
**Doncaster Victoria 3108 (AU)**
Inventor: **Bird, Graham John**
**10 Alfred Street**
**North Melbourne Victoria 3051 (AU)**
Inventor: **Houston, Timothy Leslie**
**3 Maude Street**
**Chadstone Victoria 3148 (AU)**

(74) Representative: **Beton, John Lonsdale et al**
**Imperial Chemical Industries PLC Legal**
**Department: Patents PO Box 6**
**Welwyn Garden City Herts, AL7 1HD (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to cinnoline derivatives having biological activity, in particular having herbicidal properties, to processes for the preparation of such compounds, to intermediates useful in the preparation of such compounds and processes utilizing such compounds.

Certain herbicidally active bicyclic aryloxyphenoxyalcanoic acid derivatives are disclosed in GB—A—1548847, DE—A—2745869 and FR—A—2389610.

Accordingly the invention provides a compound of formula I:

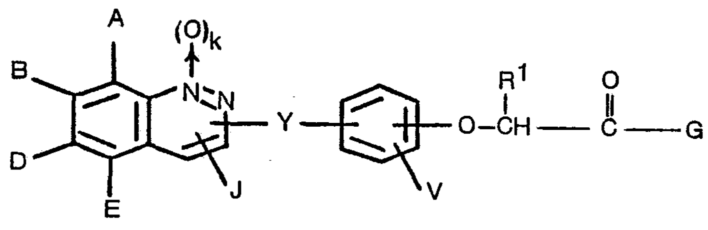

I

or a salt thereof wherein:

A, B, D, E, V and J are independently chosen from hydrogen and halogen;

$R^1$ is chosen from hydrogen and $C_1$ to $C_6$ alkyl;

G is chosen from hydroxy, $C_1$ to $C_{10}$ alkoxy, $C_2$ to $C_{10}$ alkenyloxy, $C_3$ to $C_{10}$ alkynyloxy and the group OM wherein M is an alkali metal or alkaline earth metal ion;

Y is chosen from oxygen and the group $NR^6$ wherein $R^6$ is chosen from hydrogen and $C_1$ to $C_6$ alkyl; and

k is chosen from 0 and 1.

Compounds of formula I wherein $R^1$ and $R^2$ are not the same, are optically active and the present invention also includes the individual stereo isomers of such compounds, and mixtures of those stereo isomers in addition to the racemic mixture of stereo isomers.

Particularly preferred compounds of formula I are those wherein:

A, D, E and V are hydrogen;

B is chosen from hydrogen and halogen;

J is chosen from hydrogen and halogen;

$R^1$ is methyl;

G is chosen from hydroxy, $C_1$ to $C_6$ alkoxy and the group OM wherein M is an alkali metal ion;

Y is chosen from oxygen and the group $NR^6$ wherein $R^6$ is chosen from hydrogen and methyl; and

k is chosen from 0 and 1;

those wherein:

A, D, E, J and V are hydrogen;

B is chlorine;

$R^1$ is methyl;

G is chosen from hydroxy, $C_1$ to $C_6$ alkoxy and the group OM wherein M is sodium or potassium;

Y is oxygen; and

k is 0;

and those wherein:

A, D, E, J and V are hydrogen;

B is chlorine;

$R^1$ is methyl;

G is chosen from hydroxy, $C_1$ to $C_6$ alkoxy and the group OM wherein M is sodium or potassium;

Y is the group $NR^6$ wherein $R^6$ is methyl; and

k is 0.

Examples of the compounds embraced by the invention include:

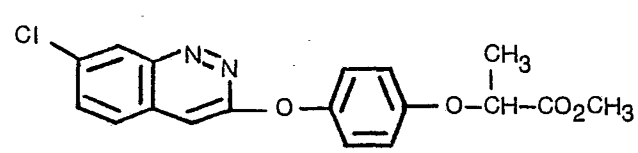

1

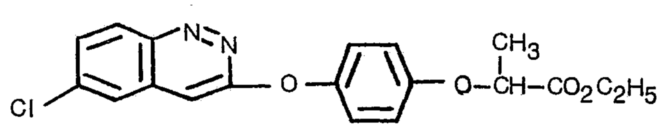

2

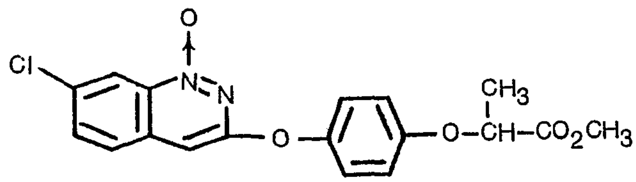

3

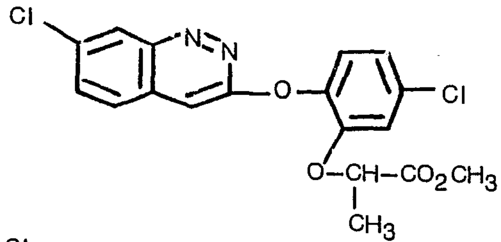

4

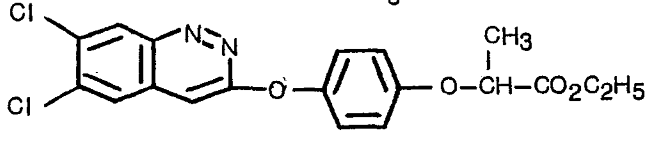

5

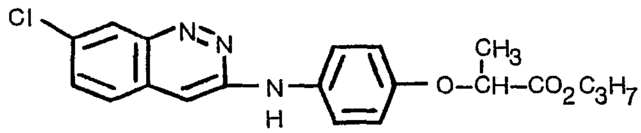

6

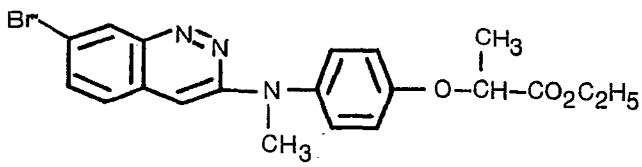

7

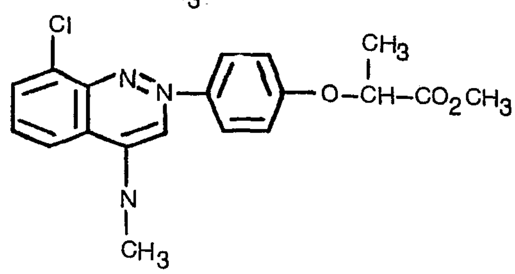

8

Preferred compounds of formula I are those 3-cinnolinyl compounds in which the phenyl ring is 1,4-substituted, that is, compounds of formula II

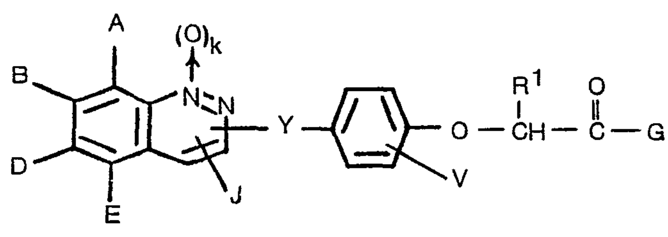

(II)

The compounds of the invention may be prepared by a variety of methods and in a further aspect the invention provides methods for the preparation of the compounds of formula I.

Compounds of formula Ia wherein G is not hydroxy may be prepared from the acid of formula Ib (I; G=OH) by, for example, neutralisation of the acid with a base to give an acid salt, or esterification of the acid with an alcohol, to give an acid ester (SCHEME A). Processes known in the art for the preparation of acid salts and acid esters may be adapted, without undue experimentation, to prepare compounds of the invention of formula Ia from compounds of the invention of formula Ib.

SCHEME A

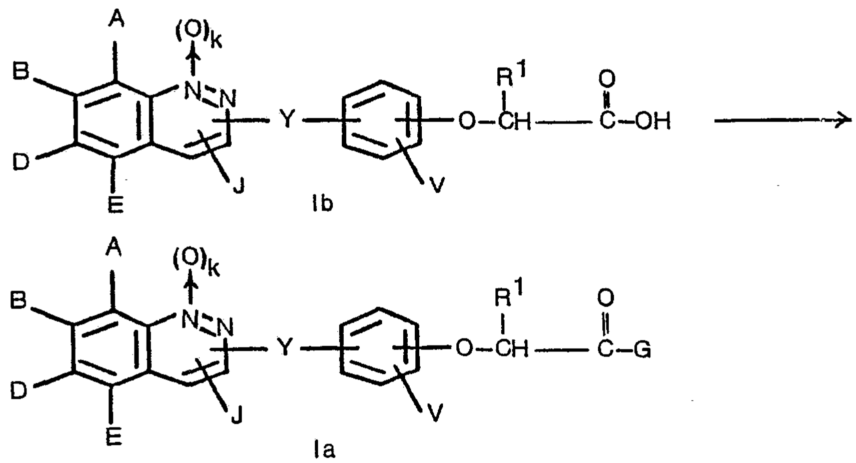

Ib

Ia

N-oxides of the invention of formula I wherein k is 1 may be prepared from compounds of the invention of formula I wherein k is 0 by oxidation. Processes known in the art for the conversion of cinnolines to cinnoline N-oxides, for example oxidations using persulfates, peroxides, peracids or peresters, may be adapted, without undue experimentation, to prepare N-oxides of the invention.

Compounds of the invention of formula I in which Y is the group $NR^6$ wherein $R^6$ is not hydrogen may be prepared from compounds of the invention of formula I in which Y is the group $NR^6$ wherein $R^6$ is hydrogen by, for example, alkylation. Processes known in the art for the preparation of derivatives of secondary amines, for example alkylations with alkyl halides may be adapted, without undue experimentation, to prepare the novel compounds of the invention wherein $R^1$ is not hydrogen.

Compounds of formula I wherein A, B, D, E, V, Y, $R^1$, J, G and k are as hereinbefore defined may be prepared by the condensation of a phenol of formula IX with a compound of formula X wherein hal is chlorine, bromine or iodine, preferably in the presence of an alkaline material, according to SCHEME B.

SCHEME B

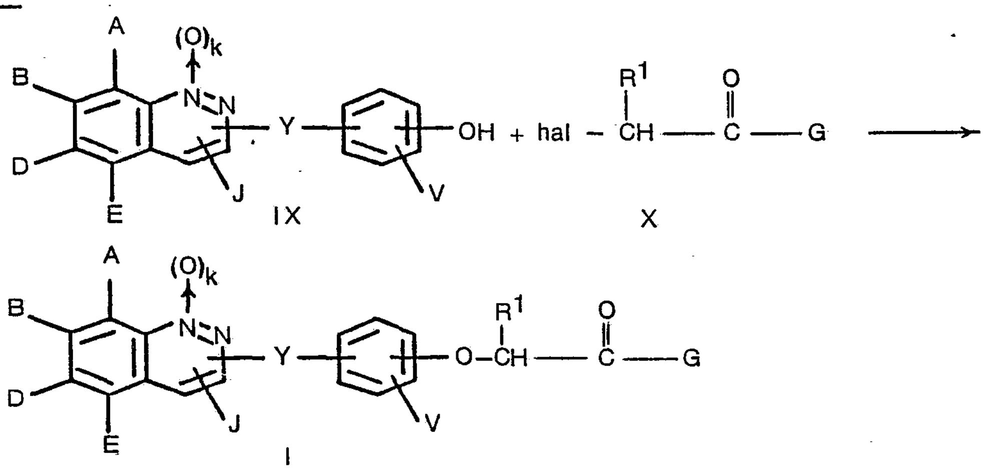

IX

X

I

Compounds of formula I may also be prepared by:

a) the condensation of the appropriate cinnoline derivatives of formula V, wherein L is a leaving group (for example, alkylsulfonyl, chlorine, bromine or iodine) with the appropriate compound of formula VI according to SCHEME C.

SCHEME C

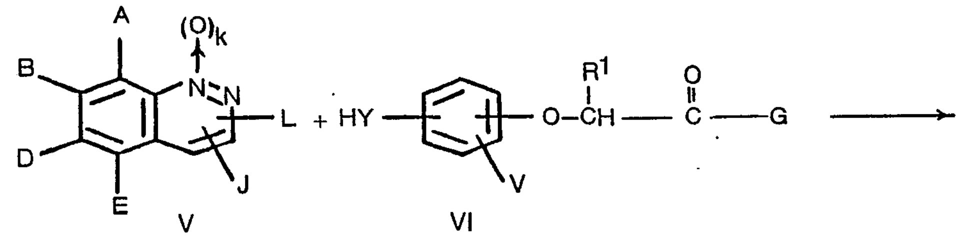

V VI

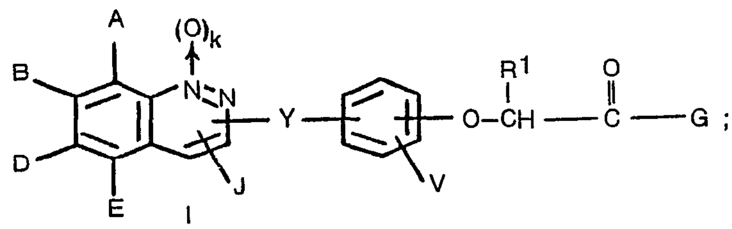

or

I

b) the following steps in sequence:

(i) the condensation of the appropriate cinnoline derivative of formula V, wherein L is a leaving group (for example, alkylsulfonyl, chlorine, bromine or iodine) with the appropriate compound of formula VII, wherein Q is hydroxy or $C_1$ to $C_6$ alkoxy to give a compound of formula VIII wherein Q is hydroxy or $C_1$ to $C_6$ alkoxy;

(ii) The dealkylation of the compound of formula VIII prepared in step (i) above wherein Q is $C_1$ to $C_6$ alkoxy to give a compound of formula IX; and

(iii) the condensation of the product of formula IX obtained in step (i) or step (ii) above with a compound of formula X according to the process described for SCHEME B above (Steps (i) and (ii) are shown in SCHEME D); or

SCHEME D

(i)

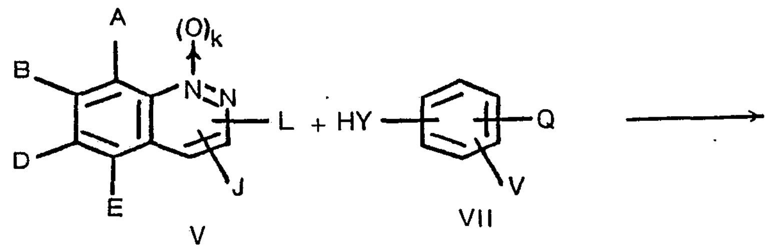

V VII

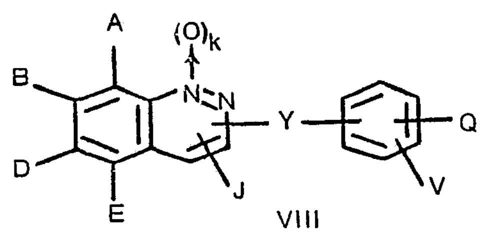

VIII

(ii)

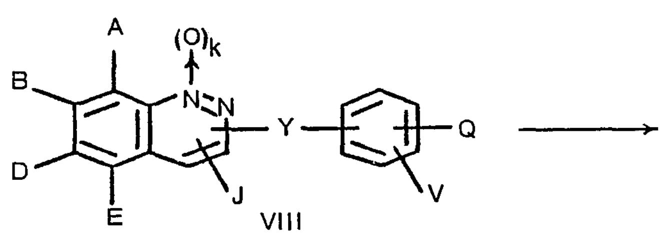

VIII

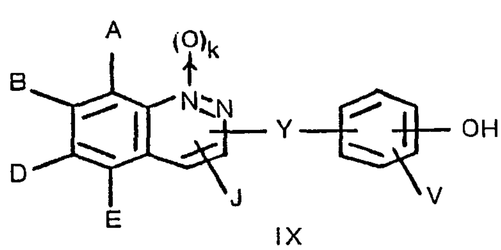

IX

c) the following steps in sequence:
(i) the condensation of the appropriate cinnoline derivative of formula XI with the appropriate benzene derivative of formula XII wherein L is a leaving group (for example, alkylsulfonyl, chlorine, bromine or iodine) and Q is hydroxy or $C_1$ to $C_6$ alkoxy to give a compound of formula VIII wherein Q is as hereinbefore defined;
(ii) the dealkylation of the compound of formula VIII prepared in step (i) above wherein Q is $C_1$ to $C_6$ alkoxy to give a compound of formula IX according to the process described for SCHEME D step (ii) above; and
(iii) the condensation of the product of formula IX obtained in step (i) or step (ii) above with a compound of formula X according to the process described for SCHEME B above (step (i) is shown in SCHEME E).

SCHEME E

(i)

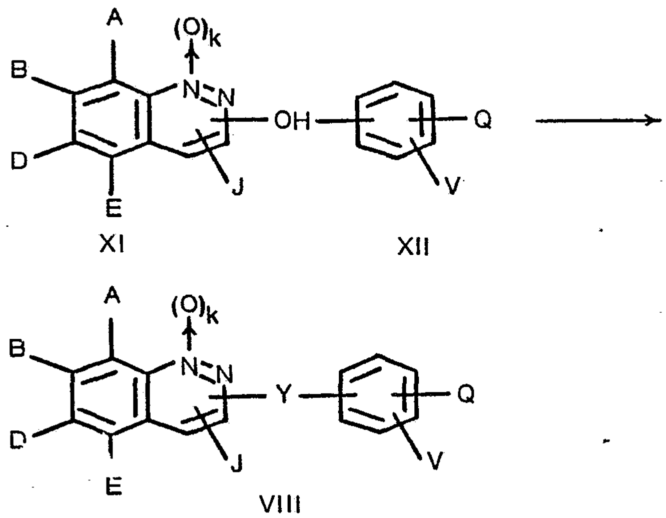

XI XII

VIII

The condensation reaction illustrated in SCHEMES B, and C to E wherein Y is oxygen and outlined above are preferably carried out in the presence of an alkaline material. Suitable alkaline materials include alkali metal and alkaline earth metal hydroxides and carbonates such as, for example, sodium hydroxide, potassium hydroxide, sodium carbonate and potassium carbonate.

The condensation reactions illustrated in Schemes B to E and outlined above are also preferably carried out in the presence of a solvent. Suitable solvents include ketones such as, for example, acetone, methyl ethyl ketone and methyl isobutyl ketone, and dipolar aprotic solvents such as, for example, dimethylformamide, dimethylacetamide, dimethylsulfoxide, *N*-methylpyrrolidone, hexamethylphosphoramide and sulfolan.

The reaction conditions required to effect the condensation reactions illustrated in SCHEMES B, C, D and E and outlined above vary according to the nature of the reactants and the solvent used. In general the reaction is facilitated by the application of heat and usually a reaction temperature in the range of 40° to 150°C and reaction time of between 0.5 and 20 hours is satisfactory. However, higher or lower reaction temperatures and/or shorter or longer reaction times may be used if desired.

The dealkylation reactions illustrated in SCHEME D and outlined in paragraphs b) (ii) and c) (ii) above may be effected using a variety of reagents known in the art. For example, aryl-alkyl ethers may be cleaved using reagents such as pyridine hydrochloride, hydroidic acid, hydrobromic acid, sodium thioethoxide in dimethylformamide, acetyl *p*-toluenesulphonate, sodium or potassium iodide in formic or acetic acid, lithium iodide in 2,4,6-collidine and boron tribromide. Reaction times and reaction conditions vary widely depending on the dealkylation agent used and the ether to be cleaved.

The reaction conditions generally employed when using the above "ether-cleavage" reagents are known to those skilled in the art and may be adapted without undue experimentation to effect the "ether-cleavage" reactions illustrated in SCHEME D and outlined in paragraph b) (ii) and c) (ii) above.

The compounds of formula VIII

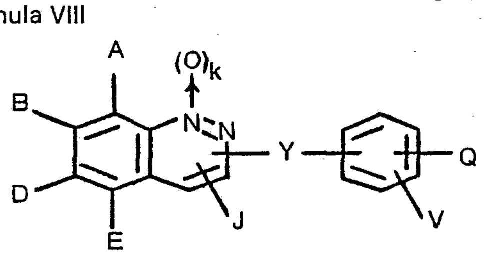

VIII

which are useful intermediates in the preparation of compounds of formula I, are novel compounds. Therefore, in a further embodiment the invention provides compounds of formula VIII wherein A, B, D, E, J, k, Y, V and Q are as hereinbefore defined.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

Generally speaking the compounds of formula I are herbicidally effective against a variety of plants. However, certain of the compounds of the invention are selectively active against monocotyledonous plants, dicotyledonous plants being relatively unaffected by rates of application of the compounds of the invention which are severely damaging or lethal to other plant species.

Moreover, certain of the compounds of formula I are selectively active within the group of monocotyledonous plants and may be used at a rate sufficient to kill or severely damage monocotyledonous weeds in a monocotyledonous cereal crop.

Therefore, in yet a further aspect the invention provides a process for selectively controlling the growth of weeds in crops which process comprises applying to the crop, or to the growth medium of the crop, a compound of formula I, as hereinbefore defined, in an amount sufficient to severely damage or kill the weeds but insufficient to damage the crop substantially.

The compounds of formula I may be applied directly to the plant (post-emergence application) or to the soil before the emergence of the plant (pre-emergence application). However, the compounds are, in general, more effective when applied to the plant post-emergence.

The compounds of formula I may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides plant growth inhibiting, plant damaging, or plant killing compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 0.01% to 90% by weight of the active ingredient. Dilute compositions ready for use preferably contain from 0.01 to 2% of active ingredient, while concentrated compositions may contain from 20 to 90% of active ingredient, although from 20 to 70% is usually preferred.

The solid compositions may be in the form of granules, or dusting powders wherein the active ingredient is mixed with a finely divided solid diluent, eg kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of a powder may be applied as foliar dusts.

Liquid compositions may comprise a solution or dispersion of an active ingredient in water optionally containing a surface-active agent, or may comprise a solution or dispersion of an active ingredient in a water-immiscible organic solvent which is dispersed as droplets in water.

Surface-active agents may be of the cationic, anionic, or non-ionic type. The cationic agents are, for example, quaternary ammonium compounds (eg cetyltrimethylammonium bromide). Suitable anionic agents are soaps; salts of aliphatic mono esters of sulphuric acid, for example sodium lauryl sulphate; and salts of sulphonated aromatic compounds, for example sodium dodecylbenzenesulphonate, sodium, calcium, and ammonium lignosulphonate, butylnaphthalene sulphonate, and a mixture of the sodium salts of diisopropyl- and triisopropylnaphthalenesulphonic acid. Suitable non-ionic agents are the condensation products of ethylene oxide with fatty alcohols such as oleyl alcohol and cetyl alcohol, or with alkylphenols such as octyl- or nonyl-phenol or octyl-cresol. Other non-ionic agents are the partial esters derived from long chain fatty acids and hexitol anhydrides, for example sorbitan monolaurate; the condensation products of the partial ester with ethylene oxide; and the lecithins.

The aqueous solutions or dispersions may be prepared by dissolving the active ingredient in water or an organic solvent optionally containing wetting or dispersing agent(s) and then, when organic solvents are used, adding the mixture so obtained to water optionally containing wetting or dispersing agent(s). Suitable organic solvents include, for example, ethylene dichloride, isopropyl alcohol, propylene glycol, diacetone alcohol, toluene, kerosene, methylnaphthalene, the xylenes and trichloroethylene.

The compositions for use in the form of aqueous solutions or dispersions are generally supplied in the form of a concentrate containing a high proportion of the active ingredient, and the concentrate is then diluted with water before use. The concentrates are usually required to withstand storage for prolonged periods and after such storage, to be capable of dilution with water to form aqueous preparations which remain homogeneous for a sufficient time to enable them to be applied by conventional spray equipment. Concentrates conveniently contain 20—90%, preferably 20—70%, by weight of the active ingredient(s). Dilute preparations ready for use may contain varying amounts of the active ingredient(s) depending upon the intended purpose; amounts of 0.01% to 10.0% and preferably 0.1% to 2%, by weight of active ingredient(s) are normally used.

A preferred form of concentrated composition comprising the active ingredient which has been finely

divided and which has been dispersed in water in the presence of a surface-active agent and a suspending agent. Suitable suspending agents are hydrophilic colloids and include, for example, polyvinylpyrrolidone and sodium carboxymethylcellulose, and the vegetable gums, for example gum acacia and gum tragacanth. Preferred suspending agents are those which impart thixotropic properties to, and increase the viscosity of the concentrate. Examples of preferred suspending agents include hydrated colloidal mineral silicates, such as montmorillonite, beidellite, nontronite, hectorite, saponite, and saucorite. Bentonite is especially preferred. Other suspending agents include cellulose derivatives and polyvinyl alcohol.

The rate of application of the compounds of the invention will depend on a number of factors including, for example, the compound chosen for use, the identity of the plants whose growth is to be inhibited the formulations selected for use and whether the compound is to be applied for foliage or root uptake. As a general guide, however, an application rate of from 0.005 to 20 kilograms per hectare is suitable while from 0.1 to 10 kilograms per hectare may be preferred.

The compositions of the invention may comprise, in addition to one or more compounds of the invention, one or more compounds not of the invention but which possess biological activity. For example, as hereinbefore indicated the compounds of the invention are in general substantially more effective against monocotyledonous plants or grass species than against dicotyledonous plants or broad-leaved species. As a result, in certain applications the herbicidal use of the compounds of the invention alone may be sufficient to protect a crop. Accordingly in yet a still further embodiment the invention provides a herbicidal composition comprising a mixture of at least one herbicidal compound of formula I as hereinbefore defined with at least one other herbicide.

The other herbicide may be any herbicide not having the formula I. It will generally be a herbicide having a complementary action. For example, one preferred class is of mixtures comprising a herbicide active against broad leaved weeds. A second preferred class is of mixtures comprising a contact herbicide.

Examples of useful complementary herbicides include:

A. benzo-2,1,3-thiadiazin-4-one-2,2-dioxides such as 3-isopropylbenzo-2,1,3-thiadiazin-4-one-2,2-dioxide (common name bentazon);

B. hormone herbicides and in particular the phenoxyalkanoic acids such as 4-chloro-2-methylphenoxy acetic acid (common name MCPA), 2-(2,4-dichlorophenoxy)propionic acid (common name dichlorprop), 2,4,5-trichlorophenoxyacetic acid (common name 2,4,5-T), 4-(4-chloro-2-methylphenoxy)butyric acid (common name MCPB), 2,4-dichlorophenoxyacetic acid (common name 2,4-D), 4-(2,4-dichlorophenoxy)butyric acid (common name 2,4-DB), 2-(4-chloro-2-methylphenoxy)propionic acid (common name mecoprop), and their derivatives (eg salts, esters, amides and the like);

C. 3-[4-(4-halophenoxy)phenyl]-1,1-dialkylureas such as 3-[4-(4-chlorophenoxy)phenyl]-1,1-dimethylurea (common name chloroxuron);

D. dinitrophenols and their derivatives (eg acetates) such as 2-methyl-4,6-dinitrophenol (common name DNOC), 2-tertiarybutyl-4,6-dinitrophenol (common name dinoterb), 2-secondarybutyl-4,6-dinitrophenol (common name dinoseb) and its ester dinoseb acetate;

E. dinitroaniline herbicides such as N',N'-diethyl-2,6-dinitro-4-trifluoromethyl-*m*-phenylenediamine (common name dinitramine), 2,6-dinitro-N,N-dipropyl-4-trifluoromethylaniline (common name trifluralin) and 4-methylsulfonyl-2,6-dinitro-N,N-dipropylaniline (common name nitralin);

F. phenylurea herbicides such as N'-(3,4-dichlorophenyl)-N,N-dimethylurea (common name diuron) and N,N-dimethyl-N'-[3-(trifluoromethyl)phenyl]urea (common name fluometuron);

G. phenylcarbamoyloxyphenylcarbamates such as 3-[(methoxycarbonyl)amino]phenyl (3-methylphenyl)-carbamate (common name phenmedipham) and 3-[(ethoxycarbonyl)amino]phenyl phenylcarbamate (common name desmedipham);

H. 2-phenylpyridazin-3-ones such as 5-amino-4-chloro-2-phenylpyridazin-3-one (common name pyrazon);

I. uracil herbicides such as 3-cyclohexyl-5,6-trimethyleneuracil (common name lenacil), 5-bromo-3-*sec*-butyl-6-methyluracil (common name bromacil) and 3-*tert*-butyl-5-chloro-6-methyluracil (common name terbacil);

J. triazine herbicides such as 2-chloro-4-ethylamino-6-(*iso*-propylamino)-1,3,5-triazine (common name atrazine), 2-chloro-4,6-di(ethylamino)-1,3,5-triazine (common name simazine) and 2-azido-4-(isopropylamino)-6-methylthio-1,3,5-triazine (common name aziprotryne);

K. 1-alkoxy-1-alkyl-3-phenylurea herbicides such as 3-(3,4-dichlorophenyl)-1-methoxy-1-methylurea (common name linuron), 3-(4-chlorophenyl)-1-methoxy-1-methylurea (common name monolinuron) and 3-(4-bromo-4-chlorophenyl)-1-methoxy-1-methylurea (common name chlorobromuron);

L. thiolcarbamate herbicides such as S-propyl dipropylthiocarbamate (common name verolate);

M. 1,2,4-triazin-5-one herbicides such as 4-amino-4,5-dihydro-3-methyl-6-phenyl-1,2,4-triazine-5-one (common name metamitron) and 4-amino-6-*tert*-butyl-4,5-dihydro-3-methylthio-1,3,4-triazin-5-one (common name metribuzin);

N. benzoic acid herbicides such as 2,3,6-trichlorobenzoic acid (common name 2,3,6-TBA), 3,6-dichloro-2-methoxybenzoic acid (common name dicamba) and 3-amino-2,5-dichlorobenzoic acid (common name chloramben);

O. anilide herbicides such as N-butoxymethyl-α-chloro-2',6'-diethylacetanilide (common name

butachlor), the corresponding N-methoxy compound (common name alachlor), the corresponding N-*iso*-propyl compound (common name propachlor) and 3′,4′-dichloropropionanilide (common name propanil);

P. dihalobenzonitrile herbicides such as 2,6-dichlorobenzonitrile (common name dichlobenil), 3,5-dibromo-4-hydroxybenzonitrile (common name bromoxynil) and 3,5-diiodo-4-hydroxybenzonitrile (common name ioxynil).

Q. haloalkanoic herbicides such as 2,2-dichloropropionic acid (common name dalapon), trichloroacetic acid (common name TCA) and salts thereof;

R. diphenylether herbicides such as 4-nitrophenyl 2-nitro-4-trifluoromethylphenyl ether (common name fluorodifen), methyl 5-(2,4-dichlorophenoxy)-2-nitrobenzoate (common name bifenox), 2-nitro-5-(2-chloro-4-trifluoromethylphenoxy)benzoic acid and 2-chloro-4-trifluoromethylphenyl 3-ethoxy-4-nitrophenyl ether and the compounds disclosed in European Patent publication No 3,416; and

S. miscellaneous herbicides including N,N-dimethyldiphenylacetamide (common name diphenamid), N-(1-naphthyl)phthalamic acid (common name naptalam) and 3-amino-1,2,4-triazole.

Examples of useful contact herbicides include:

T. bipyridylium herbicides such as those in which the active entity is the 1,1′-dimethyl-4,4′-dipyridylium ion (common name paraquat) and those in which the active entity is the 1,1′-ethylene-2,2′-dipyridylium ion (common name diquat),

U. organoarsenical herbicides such as monosodium methanearsonate (common name MSMA); and

V. amino acid herbicides such as N-(phosphonomethyl)-glycine (common name glyphosate) and its salts and esters.

The invention is now illustrated by, but in no way limited to, the following Examples.

## Example 1

Ethyl 2-{4-[4-(3-bromocinnolin-4-yl)-N-methylamino]phenoxy}propionate (17)

a) A mixture of 3-bromo-4-chlorocinnoline (0.6 g; 2.48 mmole: prepared according to the process of E. J. Alford and K. Schofield, *J. Org. Chem., 1953,* *30, 609*), 4-(N-methylamino)phenol sulfate (0.85 g, 2.48 mmole) and acetonitrile/water (1:1) was heated under reflux for a period of 1 hour. The reaction mixture was filtered and allowed to cool and on standing the product crystallised. The red crystalline product was collected by filtration, washed with water and dried to give 4-[N-(3-bromocinnolin-4-yl)-N-methylamino]-phenol (0.57 g; 70%), mp 239°C. The assigned structure was confirmed by proton magnetic resonance spectroscopy and mass spectrometry.

b) A mixture of 4-[N-(3-bromocinnolin-4-yl)-N-methylamino]phenol (1.0 g; 3.04 mmole), ethyl 2-bromopropionate (0.55 g; 3.04 mmole), anhydrous potassium carbonate (0.47 g; 3.3 mmole) and methyl ethyl ketone (30 ml) was heated under reflux. The progress of the reaction was monitored by thin layer chromatography over silica gel (eluent dichlormethane) and when the reaction was complete the reaction mixture was treated with water and the aqueous solution was extracted with dichloromethane. The dichloromethane extract was washed with water, dried over anhydrous magnesium sulfate and the solvent was removed by distillation under reduced pressure.

The residue was chromatographed over silica gel (eluent dichloromethane) to give, after removal of the solvent, ethyl 2-{4-[4-(3-bromocinnolin-4-yl)-N-methylamino]phenoxy}propionate (1.17 g, 90%) as a red oil.

The assigned structure was confirmed by proton magnetic resonance spectroscopy and mass spectrometry. Proton magnetic resonance spectrum chemical shift date (δ in ppm; $CDCl_3$): 1.3 t, 3H; 1.6, d, 3H; 3.48, s, 3H; 4.3, q, 2H; 4.7, q, 1H; 6.48—7.1, m, 4H; 7.7—8.2, m, 3H; 8.5—8.85, M, 1H.

## Example 2

Ethyl 2-{4-[7-chloro-1-oxide-cinnolin-3-yl)oxy]phenoxy}propionate (18)

a) 4-Chloro-2-amino-chloroacetophenone (12.0 g; 60 mmole; prepared from 3-chloroaniline by the method described by T. Sugasawa, M. Adachi, K. Sasakura and A. Kitagawa, *J. Org. Chem.*, *44*, 4, 1979) was dissolved in an ice-cold mixture of acetic acid (116 ml) and concentrated sulfuric acid (58 ml). An aqueous solution of 5% sodium nitrite (125 ml) was slowly added, the temperature of the reaction mixture being maintained below 5°C. On completion of the addition the mixture was stirred for a further 30 minutes, the mixture was then diluted with water and warmed to a temperature of 70—75°C. After stirring at that temperature for a period of 45 minutes the mixture was cooled and the precipitate was collected by filtration. The solid product was washed well with water and dried to give 3,7-dichloro-4-hydroxycinnoline (8.5 g), mp >260°C ($M^+$ 214).

b) A mixture of 3,7-dichloro-4-hydroxycinnoline (2.0 g) and phosphorus oxychloride (20 ml) was heated under reflux for a period of 15 minutes. The solution obtained was cooled and was poured into ice-water (100 ml).

The precipitate was collected by filtration, washed well with water, and dried to give 3,4,7-trichlorocinnoline (1.95 g). Proton magnetic resonance spectrum (δ in ppm; $CDCl_3$): 7.99, d, 1H; 8.30, d, 1H, 8.60, s, 1H.

c) *p*-Toluenesulfonylhydrazine (9.5 g) was added to a solution of 3,4,7-trichlorocinnoline (5.0 g) in chloroform (200 ml). The mixture was shaken and after standing for a period of 1 week the red-brown

precipitate was collected by filtration and dried. The solid (6.0 g) was added slowly, in portions, to an aqueous solution of sodium carbonate (14.0 g in 300 ml) heated at a temperature of 95°C. On completion of the addition the mixture was heated and stirred for a period of 4 hours after which time gas evolution had ceased. The crude product was collected by filtration and was purified by column chromatography over silica gel (eluent dichloromethane) to give 3,7-dichlorocinnoline (3.0 g) as a cream solid, mp 161°C.

d) Hydrogen peroxide (15 ml of 30% v/v) was slowly added to a stirred mixture of 3,7-dichlorocinnoline (4.0 g) and glacial acetic acid (30 ml). On completion of the addition the mixture was heated and stirred at a temperature of 60°C for a period of 6 hours and was then cooled and allowed to stand overnight at ambient temperature. The precipitate was collected by filtration and recrystallised from methanol to give 3,7-dichlorocinnoline-1-oxide (2.6 g) as fine yellow needles, mp 206°C ($M^+$ 214).

e) A mixture of 3,7-dichlorocinnoline-1-oxide (0.70 g; 3.27 mmole), ethyl 2-(4-hydroxyphenoxy)-propionate (0.69 g; 3.27 mmole), anhydrous potassium carbonate (0.50 g) and dimethylformamide (30 ml) was heated at a temperature of 100°C. The progress of the reaction was monitored by thin layer chromatography on silica gel (eluent dichloromethane). On completion of the reaction the mixture was cooled and diluted with dichloromethane. The mixture was washed well with water and the organic phase was separated and dried over anhydrous magnesium sulfate. The organic solvent was evaporated to give a yellow solid which was recrystallised from ethanol to give ethyl 2-{4-[(7-chloro-1-oxidecinnolin-3-yl)oxy]-phenoxy}propionate (0.82 g) as yellow crystals, mp 124°C.

## Example 3

Ethyl 2-{4-[(1-oxide-cinnolin-3-yl)oxy]phenoxy}propionate (19) was prepared starting from 3-bromo-4-chlorocinnoline (see Example 1) following essentially the same procedure as that described in Example 2 parts c), d) and e) Proton magnetic resonance spectrum (δ in ppm; $CDCl_3$): 1.30, t, 3H; 1.60, d, 3H; 4.20, q, 2H; 4.80, q, 1H; 7.00, m, 5H; 7.70, m, 2H; 8.50, m, 1H.

## Example 4

Ethyl 2-{4-[(7-chlorocinnolin-3-yl)oxy]phenoxy}propionate (20)

An aqueous solution of titanium trichloride (15%; 30 ml) was added slowly to a mixture of ethyl 2-{4-[(7-chloro-1-oxide-cinnolin-3-yl)oxy]phenoxy}propionate (0.4 g; 2.6 mmole), ammonium acetate (3.0 g), water (25 ml) and methanol (25 ml) stirred under a nitrogen atmosphere. On completion of the addition the mixture was stirred for a further period of one hour. The mixture was extracted with dichloromethane and the organic layer was then washed with water and dried over anhydrous magnesium sulfate. The solvent was evaporated and the residue was dissolved in benzene. Mercuric oxide (1.5 g) was added and the mixture was heated under reflux for a period of 4 hours. The mixture was cooled and filtered and the solvent was evaporated. The residue was purified by column chromatography over silica gel (eluent dichloromethane) to give ethyl 2-{4-[7-(chlorocinnolin-3-yl)oxy]phenoxy}propionate (0.27 g) as an orange oil which slowly crystallised on standing. Proton magnetic resonance spectrum (δ in ppm; $CDCl_3$): 1.30, t, 3H; 1.60, d, 3H; 4.30, q, 2H; 4.90, q, 1H; 6.90, m, 4H; 7.20, s, 1H; 7.60, m, 2H; 8.40, s, 1H.

## Example 5

Ethyl 2-{4-[N-(7-chlorocinnolin-3-yl)amino]phenoxy}propionate (21)

a) A mixture of 3-bromo-7-chlorocinnoline (2.0 g), N-acetyl-4-methoxyaniline (1.28 g), anhydrous potassium carbonate (1.25 g), cuprous oxide (0.10 g), copper powder (0.01 g) and cupric oxide (0.01 g) was heated, with stirring, at a temperature of 140—150°C for a period of 3 hours. The mixture was cooled and extracted with ethyl acetate. The ethyl acetate solution was washed with water, dried over anhydrous magnesium sulfate and the solvent was evaporated. The residue was purified by column chromatography over silica gel (eluent dichloromethane/ethyl acetate 9:1) to give N-acetyl-N-(4-methoxyphenyl)-N-(7-chlorocinnolin-3-yl)amine (0.60 g) as a dark oil. Proton magnetic resonance spectrum (δ in ppm; $CDCl_3$): 2.20, s, 3H; 3.80, s, 3H, 6.9—7.6, m, 4H; 7.80, m, 2H; 8.30, s, 1H; 8.50, s, 1H.

b) A mixture of N-acetyl-N-(4-methoxyphenyl)-N-(7-chlorocinnolin-3-yl)amine (0.60 g), glacial acetic acid (15 ml) and hydrobromic acid (15 ml of 48%) was heated under reflux for a period of eight hours. The mixture was concentrated, neutralized with aqueous sodium bicarbonate and the aqueous solution was extracted with ethyl acetate. The organic extract was washed with water, dried over anhydrous magnesium sulfate and the solvent evaporated to give 4-[N-(7-chlorocinnolin-3-yl)amino]phenol (0.50 g) as a yellow solid, mp 150°C.

c) A mixture of 4-[N-(7-chlorocinnolin-3-yl)amino]phenol (0.50 g), ethyl 2-bromopropionate (0.34 g), anhydrous potassium carbonate (0.28 g) and dimethylformamide was heated at a temperature of 120°C for a period of 4 hours. The reaction mixture was cooled and diluted with ethyl acetate. The diluted mixture was washed with water, dried over anhydrous magnesium sulfate and the solvent was evaporated to give a dark oil. The oil was purified by column chromatography over silica gel (eluent dichloromethane) to give ethyl 2-{4-[N-(7-chlorocinnolin-3-yl)amino]phenoxy}propionate (0.50 g) as a red oil. Proton magnetic resonance spectrum (δ in ppm; $CDCl_3$): 1.30, t, 3H; 1.80, d, 3H; 4.30, q, 2H; 4.90, q, 1H; 6.9—7.5, m, 7H; 8.20, s, 1H; 8.35, s, 1H.

## Example 6

Ethyl 2-{4-[N-methyl-N-(7-chlorocinnolin-3-yl)amino]phenoxy}propionate (22)

Potassium tertiarybutoxide (0.20 g) was slowly added to a stirred solution of ethyl 2-{4-[N-(7-chloro-cinnolin-3-yl)amino]phenoxy}propionate (0.50 g) in dimethylformamide. On completion of the addition methyl iodide (2.0 ml) was added and the solution was stirred for a further 15 minutes. The mixture was poured into ethyl acetate and the mixture was washed with water. The organic phase was separated, dried over anhydrous magnesium sulfate and the solvent was evaporated to give an oil. The residue was purified by column chromatography over silica gel (eluent dichloromethane) to give ethyl 2-{4-[N-methyl-N-(7-chlorocinnolin-3-yl)amino]phenoxy}propionate (0.50 g) as an oil. Proton magnetic resonance spectrum (δ in ppm; $CDCl_3$): 1.30, t, 3H;, 1.80, d, 3H; 3.70, s, 3H; 4.3, q, 2H; 4.90, q, 1H; 6.7—7.4, m, 7H; 8.2, s, 1H.

## Example 7

Ethyl 2-{4-[N-(7-chlorocinnolin-3-yl)amino]2-bromophenoxy}propionate (23)

A mixture of N-acetyl-N-(4-methoxyphenyl)-N-(7-chlorocinnolin-3-yl)amine (1.50 g), glacial acetic acid (20 ml) and hydrobromic acid (20 ml of 48%) was heated under reflux for a period of sixteen hours. The mixture was concentrated, neutralized with aqueous sodium bicarbonate and the aqueous solution was extracted with ethyl acetate. The organic extract was washed with water, dried over anhydrous magnesium sulfate and the solvent evaporated to give 2-bromo-4-[N-(7-chlorocinnolin-3-yl)amino]phenol (1.20 g) as a reddish solid, mp (decomposed) 70°C.

A mixture of 2-bromo-4-[N-(7-chlorocinnolin-3-yl)amino]phenol (1.25 g), ethyl 2-bromopropionate (0.65 g), anhydrous potassium carbonate (0.55 g) and methyl ethyl ketone was heated under reflux for 12 hours. The reaction mixture was cooled and diluted with ethyl acetate. The diluted mixture was washed with water, dried over anhydrous magnesium sulfate and the solvent was evaporated to give a dark oil. The oil was purified by column chromatography over silica gel (eluant dichloromethane/acetone 9:1) to give ethyl 2-{4-[N-(7-chlorocinnolin-3-yl)amino]2-bromophenoxy}propionate (1.10 g) as an oil. Proton magnetic resonance spectrum (δ in ppm; $CDCl_3$): 1.30, t, 3H; 1.70, d, 3H; 4.20, q, 2H; 4.80, q, 1H; 6.80, s, 1H; 7.00—8.60, m, 5H; 8.20, s, 1H.

## Example 8

Ethyl 2-{4-[N-methyl-N-(7-chlorocinnolin-3-yl)amino]-2-bromophenoxy}propionate (24)

Sodium hydride (0.03 g) was added to a stirred solution of ethyl 2-{4-[N-(7-chlorocinnolin-3-yl)amino]-2-bromophenoxy}propionate (0.40 g) in dimethylformamide. The mixture was stirred for 2 minutes, then methyl iodide (1 ml) was added and stirring was continued for 15 minutes. The mixture was poured into ethyl acetate and the mixture was washed with water. The organic phase was separated, dried over anhydrous magnesium sulfate and the solvent was evaporated to give an oil. The residue was purified by column chromatography over silica gel (eluant dichloromethane/acetone, 9:1) to give ethyl 2-{4-[N-methyl-N-(7-chlorocinnolin-3-yl)amino]2-bromophenoxy}propionate (0.23 g) as an oil.

Proton magnetic resonance spectrum (δ in ppm; $CDCl_3$): 1.30, t, 3H; 1.70, d, 3H; 3.7, s, 3H; 4.2, q, 2H; 4.8, q, 1H; 6.8, s, 1H; 7.0—8.0, m, 5H; 8.2, s, 1H.

## Example 9

Ethyl 2-{4-[(4,7-dichlorocinnolin-3-yl)oxy]phenoxy}propionate (25)

A mixture of ethyl 2-{4-[7-chloro-1-oxidecinnolin-3-yl)oxy]phenoxy}propionate (0.70 g) and phosphorus oxychloride (20 ml) was heated at 100°C for 1 hr. The mixture was cooled, poured into ice-water and extracted with dichloromethane. The dichloromethane extract was washed with water, dried over anhydrous magnesium sulfate and evaporated to give a crude oil. The residue was purified by column chromatography over silica gel (eluant dichloromethane) to give ethyl 2-{4-[(4,7-dichlorocinnolin-3-yl)oxy]-phenoxy}propionate (0.080 g) as an oil. Proton magnetic resonance spectrum (δ in ppm; $CDCl_3$): 1.3, t, 3H; 1.7, d, 3H; 4.3, q, 2H; 4.7, q, 1H; 6.9—7.3, m, 4H; 7.6—8.1, m, 2H; 8.4, s, 1H.

## Example 10

Concentrated formulations of the compounds of the invention were prepared by:

a) in the case of oils and waxy solids, dissolving the compound in toluene containing 7% v/v "Teric" N13 ("Teric" is a Trade Mark and "Teric" N13, a product of ethoxylation of nonylphenol, is available from ICI Australia Limited) and 3% v/v "Kemmat" SC15B ("Kemmat" is a Trade Mark and "Kemmat" SC15B is a formulation of calcium dodecylbenzene sulfonate); or

b) in the case of crystalline solids, adding 5 parts by weight of the compound and 1 part by weight of "Dyapol" PT ("Dyapol" is a Trade Mark and "Dyapol" PT is an anionic suspending agent) to 94 parts by weight of an aqueous solution containing 0.25% v/v of "Teric" N8 (a product of ethoxylation of nonylphenol) and ball-milling the mixture to produce a stable suspension. The emulsifiable concentrates and suspensions were then diluted with water to give an aqueous composition of the required concentration suitable for use in the evaluation of the pre-emergence and post-emergence herbicidal activity of the compounds of the invention.

Example 11

The pre-emergent herbicidal activity of the compounds of the invention formulated as described in Example 10 was assessed by the following procedure.

The seeds of the test species were sown in rows 2 cm deep in soil contained in need boxes. The monocotyledonous plants and the dicotyledonous plants were sown in separate boxes and after sowing the two boxes were sprayed with the required quantity of a composition of the invention. Two duplicate seed boxes were prepared in the same manner but were not sprayed with a composition of the invention and were used for comparison purposes. All the boxes were placed in a glasshouse, lightly watered with an overhead spray to initiate germination and then subirrigated as required for optimum plant growth. After three weeks the boxes were removed from the glasshouse and the effect of the treatment was visually assessed. The results are presented in Table 1 where the damage to plants is rated on a scale of from 0 to 3 where 0 represents from 0 to 25% damage, 3 represents 75 to 99% kill and 3+ represents 100% kill.

A dash (—) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 1

PRE-EMERGENCE HERBICIDAL ACTIVITY

| Compound No | APPLICATION Rate (kg/ha) | TEST PLANT | | | | | | | |
|---|---|---|---|---|---|---|---|---|---|
| | | Wh | Ot | Rg | Jm | P | Ip | Ms | Sf |
| 18 | 5.0 | 2 | 3 | 3+ | 3+ | 0 | 0 | 0 | 0 |
| 18 | 1.0 | 0 | 0 | 0 | 3 | 0 | 0 | 0 | 0 |
| 20 | 5.0 | 1 | 3+ | 3+ | 3+ | 0 | 0 | 0 | 0 |
| 20 | 1.0 | 0 | 0 | 1 | 2 | 0 | 0 | 0 | 0 |
| 20 | 0.5 | 0 | 0 | 0 | 2 | 0 | 0 | 0 | 0 |
| 20 | 0.25 | 0 | 0 | 0 | 0 | 0 | 0 | 0 | 0 |
| 22 | 1.0 | 0 | 0 | 3 | 3 | 0 | 0 | 0 | 0 |
| 22 | 0.5 | 0 | 0 | 2 | 3 | 0 | 0 | 0 | 0 |
| 22 | 0.25 | 0 | 0 | 0 | 1 | 0 | 0 | 0 | 0 |
| 24 | 5.0 | 0 | 0 | 2 | 2 | 0 | 0 | 0 | 0 |
| 25 | 5.0 | 0 | 0 | 0 | 0 | 0 | 0 | 0 | 0 |
| 25 | 1.0 | 0 | 0 | 0 | 0 | 0 | 0 | 0 | 0 |

Example 12

The post-emergent herbicidal activity of the compounds of the invention formulated as described in Example 10 was assessed by the following procedure.

The seeds of the test species were sown in rows 2 cm deep in soil contained in seed boxes. The monocotyledonous plants and the dicotyledonous plants were sown in separate seed boxes in duplicate. The four seed boxes were placed in a glasshouse, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glasshouse and sprayed with the required quantity of a composition of the invention. After spraying the boxes were returned to the glasshouse for further 3 weeks and the effects of treatment was visually assessed by comparison with the untreated controls. The results are presented in Table 2 where the damage to plants is rated on a scale of from 0 to 3 where 0 represents 0 to 25% damage. 3 represents 75 to 99% kill and 3+ represents 100% kill. A dash (—) means that no experiment was carried out.

The names of the test plants are as follows:

| | |
|---|---|
| Wh | Wheat |
| Ot | Wild Oats |
| Rg | Ryegrass |
| Jm | Japanese millet |
| P | Peas |
| Ip | Ipomea |
| Ms | Mustard |
| Sf | Sunflower |

TABLE 2

POST-EMERGENCE HERBICIDAL ACTIVITY

| Compound No | APPLICATION Rate (kg/ha) | TEST PLANT | | | | | | | |
|---|---|---|---|---|---|---|---|---|---|
| | | Wh | Ot | Rg | Jm | P | Ip | Ms | Sf |
| 18 | 5.0 | 3+ | 3+ | 3+ | 3+ | 0 | 0 | 0 | 0 |
| 18 | 1.0 | 3 | 1 | 3 | 3+ | 0 | 0 | 0 | 0 |
| 20 | 5.0 | 3+ | 3+ | 3+ | 3+ | 1 | 3+ | 3 | 2 |
| 20 | 1.0 | 3+ | 3+ | 3+ | 3+ | 0 | 1 | 2 | 1 |
| 20 | 0.5 | 3 | 3+ | 3 | 3+ | 0 | 0 | 0 | 0 |
| 20 | 0.25 | 3 | 3 | 2 | 3+ | 0 | 0 | 0 | 0 |
| 22 | 1.0 | 3+ | 3+ | 3+ | 3+ | 0 | 0 | 0 | 0 |
| 22 | 0.5 | 3+ | 3+ | 3+ | 3+ | 0 | 0 | 0 | 0 |
| 22 | 0.25 | 3+ | 3 | 3+ | 3+ | 0 | 0 | 0 | 0 |
| 24 | 5.0 | 2 | 2 | 3 | 3+ | 0 | 0 | 0 | 0 |
| 25 | 5.0 | 3 | 2 | 0 | 3 | 0 | 0 | 0 | 0 |
| 25 | 1.0 | 1 | 0 | 0 | 2 | 0 | 0 | 0 | 0 |

Example 13

The compounds were formulated for test by mixing an appropriate amount with 5 ml of an emulsion prepared by diluting 160 ml of a solution containing 21.8 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to young pot plants (post-emergence test) of the species named in Table 3 below. Damage to test plants was assessed after 14 days on a scale of 0 to 5 where 0 is 0 to 20% damage and 5 is complete kill. In a test for pre-emergence herbicidal activity, seeds of the test plants were sown in a shallow slit formed in the surface of soil in fibre trays. The surface was then levelled and sprayed, and fresh soil then spread thinly over the sprayed surface. Assessment of herbicidal damage was carried out after 21 days using the same scale of 0 to 5 as the post-emergence test. In both cases the degree of herbicidal damage was assessed by comparison with untreated control plants. The results are given in Table 3 below. A dash (—) means that no experiment was carried out.

The names of the test plants were as follows:

| | |
|---|---|
| Sb | Sugar beet |
| Rp | Rape |
| Ct | Cotton |
| Sy | Soybean |
| Mz | Maize |
| Ww | Winter wheat |
| Rc | Rice |
| Sn | *Senico vulgaris* |
| Ip | *Ipomea purpurea* |
| Am | *Amaranthus retroflexus* |
| Pi | *Polygonum aviculare* |
| Ca | *Chenopodium album* |
| Ga | *Galium aparine* |
| Xa | *Xanthium pensylvanicum* |
| Ab | *Abutilon theophrasti* |
| Cv | *Convolvulus arvensis* |
| Av | *Avena fatua* |
| Dg | *Digitaria sanguinalis* |
| Al | *Alopecurus myosuroides* |
| St | *Setaria viridis* |
| Ec | *Echinochloa crus-galli* |
| Sh | *Sorghum halepense* |
| Ag | *Agropyron repens* |
| Cn | *Cyperus rotundas* |

TABLE 3 PART A

| Compound No | APPLICATION Method Rate (kg/ha) | TEST PLANT | | | | | | | | | | | |
|---|---|---|---|---|---|---|---|---|---|---|---|---|---|
| | | Sb | Rp | Ct | Sy | Mz | Ww | Rc | Sn | Ip | Am | Pi | Ca |
| 18 | PRE 0.2 | 0 | 0 | 0 | 0 | 2 | 2 | 3 | 0 | 0 | 0 | 0 | |
| 18 | PRE 0.05 | 0 | 0 | 0 | 0 | 0 | 0 | 0 | 0 | 0 | 0 | 0 | 0 |
| 18 | POST 0.2 | 0 | 0 | 0 | 0 | 5 | 4 | 3 | 0 | 0 | — | 0 | 0 |
| 18 | POST 0.05 | 0 | 0 | 0 | 0 | 4 | 3 | 2 | 0 | 0 | — | 0 | 0 |
| 19 | PRE 0.5 | 3 | 0 | 0 | 1 | 3 | 2 | 4 | 0 | 0 | 0 | 0 | 0 |
| 19 | POST 0.5 | 2 | 0 | 0 | 0 | 4 | 2 | 4 | 1 | 0 | — | 0 | 0 |
| 20 | PRE 0.5 | 0 | 0 | 0 | 1 | 3 | 2 | 5 | 0 | 1 | 0 | 0 | 0 |
| 20 | PRE 0.2 | — | 0 | 0 | 0 | 1 | 5 | 4 | 0 | 0 | 0 | 0 | 0 |
| 20 | PRE 0.05 | 0 | 0 | 0 | 0 | 0 | 0 | 0 | 0 | — | 0 | 0 | 0 |
| 20 | POST 0.5 | 2 | 0 | 1 | 0 | 5 | 4 | — | 1 | 0 | — | 0 | 0 |
| 20 | POST 0.2 | 2 | 1 | 0 | 0 | 3 | 3 | — | 0 | 0 | — | 0 | 0 |
| 20 | POST 0.05 | 0 | — | 0 | 0 | 4 | 3 | 1 | 0 | 0 | — | 0 | 0 |
| 22 | PRE 1.0 | 0 | 0 | 0 | 0 | 0 | 3 | 3 | 1 | 1 | — | 0 | 0 |
| 22 | POST 1.0 | 0 | 0 | 0 | 0 | 5 | 4 | 4 | 0 | 0 | 1 | 0 | 0 |

TABLE 3 PART B

| Compound No | APPLICATION Method Rate (kg/ha) | TEST PLANT | | | | | | | | | | | |
|---|---|---|---|---|---|---|---|---|---|---|---|---|---|
| | | Ga | Xa | Ab | Cv | Av | Dg | Al | St | Ec | Sh | Ag | Cn |
| 18 | PRE 0.2 | 0 | 0 | 0 | 0 | 2 | 4 | 4 | 5 | 3 | 2 | 2 | 0 |
| 18 | PRE 0.05 | 0 | 0 | 0 | 0 | 0 | 3 | 1 | 0 | 0 | 0 | 0 | 0 |
| 18 | POST 0.2 | 0 | 0 | 0 | 0 | 4 | 4 | 4 | 4 | 5 | 4 | 1 | 0 |
| 18 | POST 0.05 | 0 | 0 | 0 | 1 | 2 | 4 | 2 | 4 | 3 | 3 | 0 | 0 |
| 19 | PRE 0.5 | 1 | 1 | 0 | 0 | 0 | 2 | 5 | 1 | 5 | 3 | 1 | 1 |
| 19 | POST 0.5 | 0 | 1 | 1 | 0 | 0 | 2 | 3 | 3 | 4 | 4 | 1 | 1 |
| 20 | PRE 0.5 | 2 | 0 | 0 | 0 | 3 | 4 | 5 | 5 | 5 | 5 | 5 | 3 |
| 20 | PRE 0.2 | 1 | 0 | 0 | 0 | 4 | 3 | 4 | 5 | 5 | 3 | 5 | 0 |
| 20 | PRE 0.05 | 0 | 0 | 0 | 0 | 0 | 0 | 0 | 0 | 0 | 0 | 0 | 0 |
| 20 | POST 0.5 | 0 | 0 | 0 | 0 | 4 | 4 | 3 | 4 | 4 | 4 | — | 0 |
| 20 | POST 0.2 | 0 | — | 0 | 0 | 4 | 4 | 3 | 4 | 4 | 4 | — | 0 |
| 20 | POST 0.05 | 2 | 0 | 0 | 3 | 3 | 4 | 3 | 3 | 3 | 2 | 0 | 0 |
| 22 | PRE 1.0 | 1 | 0 | 0 | 0 | 3 | 4 | 5 | 3 | 0 | 3 | 5 | 0 |
| 22 | POST 1.0 | 0 | 0 | 0 | 0 | 4 | 5 | 4 | 5 | 5 | 5 | 4 | 2 |

**Claims**

1. A compound of formula I:

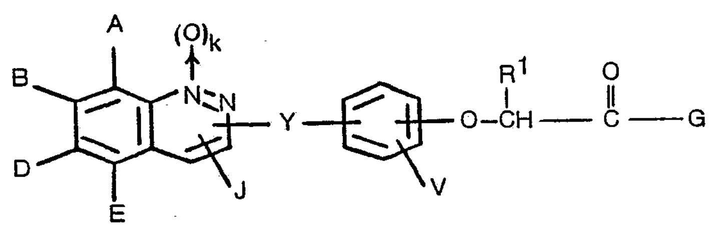

I

or a salt thereof wherein:

A, B, D, E, V and J are independently chosen from hydrogen and halogen;

$R^1$ is chosen from hydrogen and $C_1$ to $C_6$ alkyl;

G is chosen from hydroxy, $C_1$ to $C_{10}$ alkoxy, $C_2$ to $C_{10}$ alkenyloxy, $C_3$ to $C_{10}$ alkynyloxy and the group OM wherein M is an alkali metal or alkaline earth metal ion;

Y is chosen from oxygen and the group $NR^6$ wherein $N^6$ is chosen from hydrogen and $C_1$ to $C_6$ alkyl; and

k is chosen from 0 and 1.

2. A compound according to claim 1 of formula II

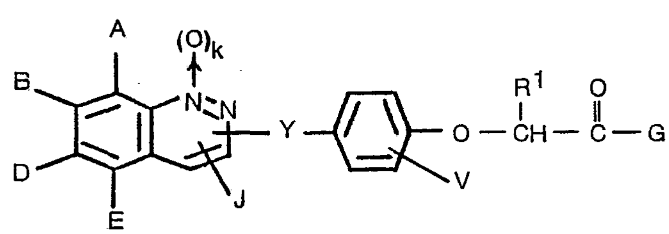

(II)

3. A compound according to claim 1 or 2 wherein:
A, D, E and V are hydrogen;
B is chosen from hydrogen and halogen;
J is chosen from hydrogen and halogen;
$R^1$ is methyl;
G is chosen from hydroxy, $C_1$ to $C_6$ alkoxy and the group OM wherein M is an alkali metal ion;
Y is chosen from oxygen and the group $NR^6$ wherein $R^6$ is chosen from hydrogen and methyl; and
k is chosen from 0 and 1.

4. A compound according to claim 1, 2 or 3 wherein:
A, D, E, J and V are hydrogen;
B is chlorine;
$R^1$ is methyl;
G is chosen from hydroxy, $C_1$ to $C_6$ alkoxy and the group OM wherein M is sodium or potassium;
Y is oxygen; and
k is 0.

5. A compound according to claim 1, 2 or 3 wherein:
A, D, E, J and V are hydrogen;
B is chlorine;
$R^1$ is methyl;
G is chosen from hydroxy, $C_1$ to $C_6$ alkoxy and the group OM wherein M is sodium or potassium;
Y is the group $NR^6$ wherein $R^6$ is methyl; and
k is 0.

6. A compound which is ethyl 2-(4-[7-chlorocinnolin-3-yl)oxy]phenoxy)propionate or ethyl 2(4-[N-(7-chlorocinnolin-3-yl)-N-methylamino]phenoxy)propionate.

7. A compound of formula VIII

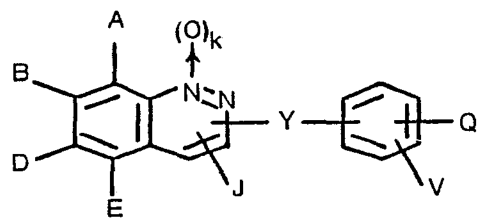

VIII

wherein A, B, D, E, J, V, Y and k are as defined in any one of claims 1 to 5 and Q is chosen from hydroxy and $C_1$ to $C_6$ alkoxy.

8. A herbicidal composition comprising as active ingredient a compound as claimed in any one of claims 1 to 6 and a carrier therefor.

9. A process for severely damaging or killing unwanted plants which process comprises applying to said plants, or to the growth medium of said plants, an effective amount of a compound as claimed in any one of claims 1 to 6 or a composition as claimed in claim 8.

10. A process for selectively controlling the growth of monocotyledonous weeds in dicotyledonous crops which process comprises applying to said crop, or to the growth medium of said crop, a compound as claimed in any one of claims 1 to 6 or a composition as claimed in claim 8 in an amount sufficient to severely damage or kill the weeds but insufficient to substantially damage the crop.

11. A process according to claim 9 or 10 wherein the compound is applied at a rate in the range from 0.005 to 20 kilograms per hectare.

12. A process for the synthesis of a compound of formula I as claimed in any one of claims 1 to 6 which process comprises either the reaction of a cinnoline derivative of formula IX with a compound of formula X:

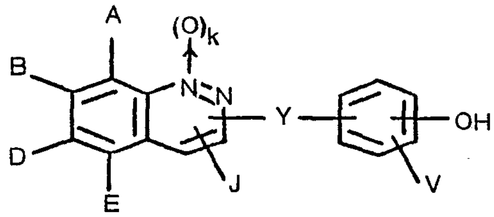

IX

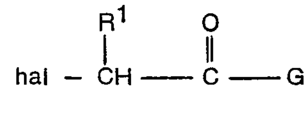

X

wherein A, B, D, E, J, V, Y, k, $R^1$ and G are as defined in any one of claims 1 to 5 and hal is chlorine, bromine or iodine,
or the reaction of a cinnoline derivative of formula V with a compound of formula VI

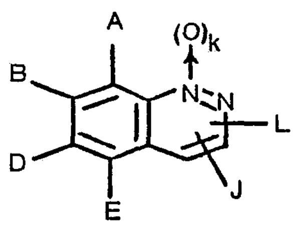 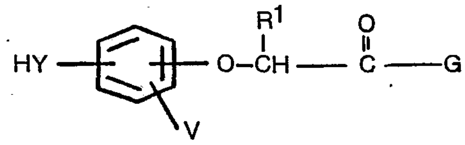

V VI

wherein A, B, D, E, J, V, Y, k, $R^1$ and G are as defined in any one of claims 1 to 5 and L is a leaving group.

13. A process according to claim 12 wherein the cinnoline compound of formula IX is prepared by reacting a cinnoline derivative of formula V as defined in claim 12 with a compound of formula VII:

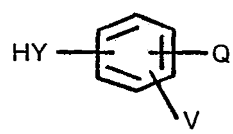

VII

wherein Y and V are as defined in any one of claims 1 to 5 and Q is hydroxy or $C_1$ to $C_6$ alkoxy, to give a compound of formula VIII,

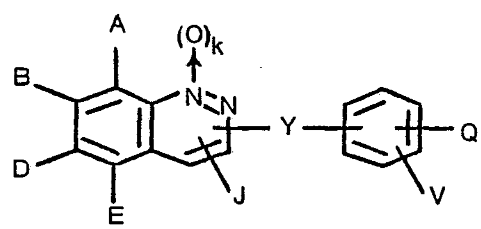

VIII

wherein A, B, D, E, J, V, Y and k are as defined in any one of claims 1 to 5 and Q is as defined above, and, when Q is $C_1$ to $C_6$ alkoxy, dealkylating the compound of formula VIII to give the compound of formula IX as defined in claim 12.

**Patentansprüche**

1. Verbindung der Formel I

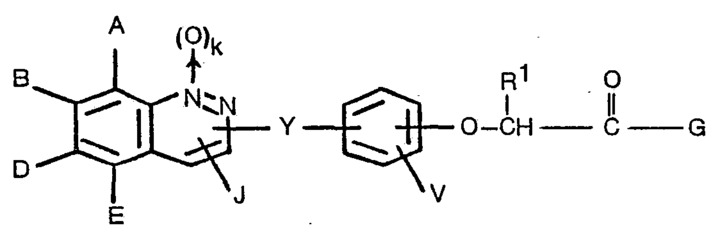

(I)

oder ein Salz davon, worin:

A, B, D, E, V und J unabhängig ausgewählt sind aus Wasserstoff und Halogen;

$R^1$ ausgewählt ist aus Wasserstoff und $C_1$—$C_6$-Alkyl;

G ausgewählt ist aus Hydroxy, $C_1$—$C_{10}$-Alkoxy, $C_2$—$C_{10}$-Alkenyloxy, $C_3$—$C_{10}$-Alkinyloxy und der Gruppe OM, worin M ein Alkalimetall- oder Erdalkalimetallion ist;

Y ausgewählt ist aus Sauerstoff und der Gruppe $NR^6$, worin $R^6$ ausgewählt ist aus Wasserstoff und $C_1$—$C_6$-Alkyl; und

k ausgewählt ist aus 0 und 1.

2. Verbindung nach Anspruch 1 der Formel II

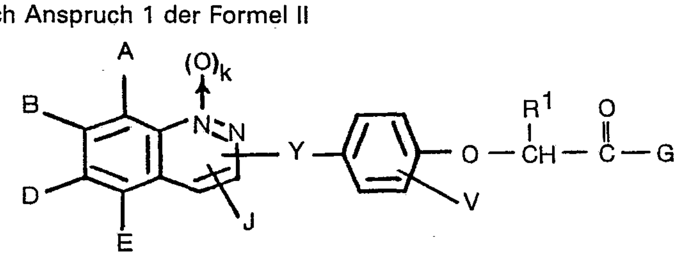

(II)

3. Verbindung nach Anspruch 1 oder 2, worin:
A, D, E und V für Wasserstoff stehen;
B ausgewählt ist aus Wasserstoff und Halogen;
J ausgewählt ist aus Wasserstoff und Halogen;
$R^1$ für Methyl steht;
G ausgewählt ist aus Hydroxy, $C_1$—$C_6$-Alkoxy und der Gruppe OM, worin M ein Alkalimetallion ist;
Y ausgewählt ist aus Sauerstoff und der Gruppe $NR^6$, worin $R^6$ ausgewählt ist aus Wasserstoff und Methyl; and
k ausgewählt ist aus 0 und 1.

4. Verbindung nach Anspruch 1, 2 oder 3, worin:
A, D, E, J und V für Wasserstoff stehen;
B für Chlor steht;
$R^1$ für Methyl steht;
G ausgewählt ist aus Hydroxy, $C_1$—$C_6$-Alkoxy und der Gruppe OM, worin M Natrium oder Kalium ist;
Y fur Sauerstoff steht; und
k für 0 steht.

5. Verbindung nach Anspruch 1, 2 oder 3, worin:
A, D, E, J und V für Wasserstoff stehen;
B für Chlor steht;
$R^1$ für Methyl steht;
G ausgewählt ist aus Hydroxy, $C_1$—$C_6$-Alkoxy und der Gruppe OM, worin M Natrium oder Kalium ist;
Y für die Gruppe $NR^6$ steht, worin $R^6$ für Methyl steht; und
k für 0 steht.

6. Verbindung, welche der 2-(4-[(7-Chlorocinnolin-3-yl)oxy]phenoxy)propionsäure-ethylester oder der 2-(4-[N-(7-Chlorocinnolin-3-yl)-N-methylamino]phenoxy)propionsäure-ethylester ist.

7. Verbindung der Formel VIII

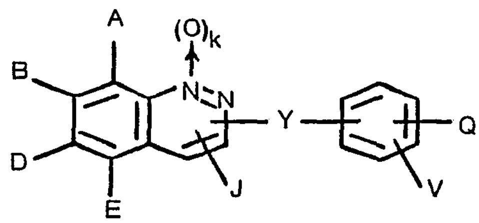

VIII

worin A, B, D, E, J, V, Y und k die in einem der Ansprüche 1 bis 5 angegebenen Bedeutungen besitzen und Q ausgewählt ist aus Hydroxy und $C_1$—$C_6$-Alkoxy.

8. Herbizide Zusammensetzung, welche als aktiven Bestandteil eine Verbindung nach einem der Ansprüche 1 bis 6 und einen Träger hierfür enthält.

9. Verfahren zum starken Schädigen oder Abtöten von unerwünschten Pflanzen, bei welchem auf die Pflanzen oder auf das Wachstumsmedium der Pflanzen eine wirksame Menge einer Verbindung nach einem der Ansprüche 1 bis 6 oder einer Zusammensetzung nach Anspruch 8 aufgebracht wird.

10. Verfahren zur selektiven Beeinflussung des Wachstums von einkeimblättrigen Ungräsern in zweikeimblättrigen Anpflanzungen, bei welchem Verfahren auf die Anpflanzung oder auf das Wachstumsmedium der Anpflanzung eine Verbindung nach einem der Ansprüche 1 bis 6 oder eine Zusammensetzung nach Anspruch 8 in einer Menge aufgebracht wird, die ausreicht, die Ungräser stark zu schädigen oder abzutöten, die aber nicht ausreicht, die Anpflanzung wesentlich zu schädigen.

11, Verfahren nach Anspruch 9 oder 10, bei welchem die Verbindung in einer Rate im Bereich von 0,005 bis 20 kg/ha aufgebracht wird.

12. Verfahren zur Synthese einer Verbindung der Formel I nach einem der Ansprüche 1 bis 6, bei welchem entweder ein Cinnolinderivat der Formel IX mit einer Verbindung der Formel X

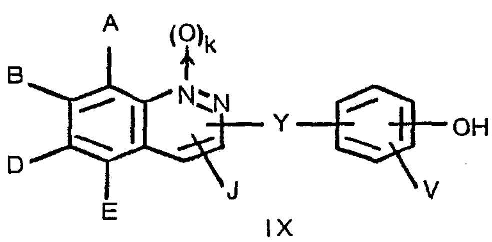

IX

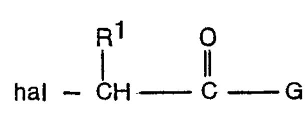

X

worin, A, B, D, E, J, V, Y, k, $R^1$ und G die in einem der Ansprüche 1 bis 5 angegebene Bedeutung besitzen und hal für Chlor, Brom oder Jod steht, oder ein Cinnolinderivat der Formel V mit einer Verbindung der Formel VI

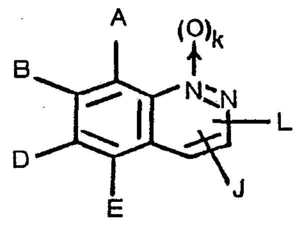

V

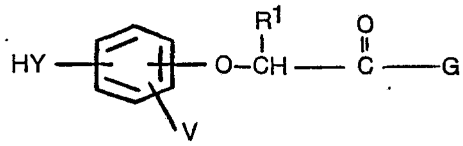

VI

worin A, B, D, E, J, V, Y, k, $R^1$ und G die in einem der Ansprüche 1 bis 5 angegebene Bedeutung besitzen und L für eine abspaltbare Gruppe steht, umgesetzt wird.

13. Verfahren nach Anspruch 12, bei welchem die Cinnolinverbindung der Formel IX dadurch hergestellt wird, daß ein Cinnolinderivat der Formel V nach anspruch 12 mit einer Verbindung der Formel VII

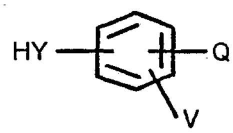

VII

worin Y und V die in einem der Ansprüche 1 bis 5 angegebene Bedeutung besitzen und Q für Hydroxy oder $C_1$—$C_6$-Alkoxy steht, umgesetzt wird, wobei eine Verbindung der Formel VIII

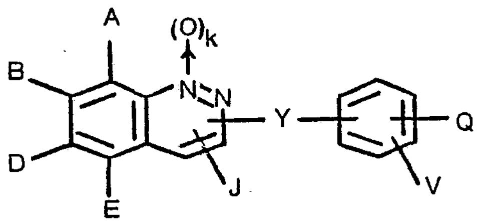

VIII

worin A, B, D, E, J, V, Y und k die in einem der Ansprüche 1 bis 5 angegebene Bedeutung besitzen und Q die oben angegebene Bedeutung besitzt, entsteht, und, wenn Q für $C_1$—$C_6$-Alkoxy steht, die Verbindung der Formel VIII dealkyliert wird, wobei eine Verbindung der Formel IX nach Anspruch 12 entsteht.

**Revendications**

1. Composé de formule I:

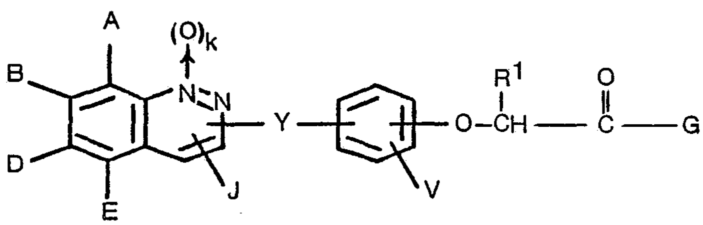

(I)

ou un sel de ce composè, formule dans laquelle:

A, B, D, E, V et J sont choisis indépendamment entre l'hydrogène et un halogène;

$R^1$ est choisi entre l'hydrogène et un groupe alkyle en $C_1$ à $C_6$;

G est choisi entre un groupe hydroxy, alkoxy en $C_1$ à $C_{10}$, alcényloxy en $C_2$ à $C_{10}$, alcynyloxy en $C_3$ à $C_{10}$ et le groupe OM dans lequel M est un ion de métal alcalin ou de métal alcalino-terreux;

Y est choisi entre l'oxygène et le groupe $NR^6$ dans lequel

$R^6$ est choisi entre l'hydrogène et un groupe alkyle en $C_1$ à $C_6$; et

k est choisi entre 0 et 1.

2. Composé suivant la revendication 1, de formule II

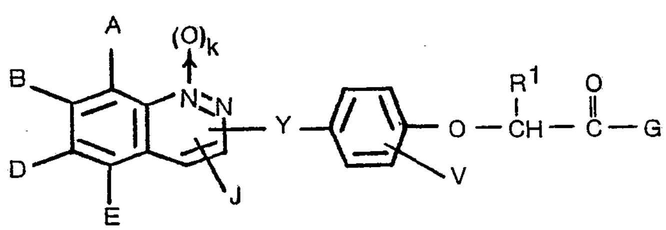

(II)

3. Composé suivant la revendication 1 ou 2, dans lequel:
A, D, E et V représentent l'hydrogène;
B est choisi entre l'hydrogène et un halogène;
J est choisi entre l'hydrogène et un halogène;
$R^1$ est un groupe méthyle;
G est choisi entre un groupe hydroxy, alkoxy en $C_1$ à $C_6$ et le groupe OM dans lequel M est un ion de métal alcalin;
Y est choisi entre l'oxygène et le groupe $NR^6$ dans lequel $R^6$ est choisi entre l'hydrogène et le groupe méthyle; et
k est choisi entre 0 et 1.

4. Composé suivant la revendication 1, 2 ou 3, dans lequel:
A, D, E, J et V sont de l'hydrogène;
B est du chlore;
$R^1$ est le groupe méthyle;
G est choisi entre un groupe hydroxy, alkoxy en $C_1$ à $C_6$ et le groupe OM dans lequel M est le sodium ou le potassium;
Y est l'oxygène; et
k est égal à 0.

5. Composé suivant la revendication 1, 2 ou 3, dans lequel:
A, D, E, J et V sont de l'hydrogène;
B est du chlore;
$R^1$ est le groupe méthyle;
G est choisi entre un groupe hydroxy, alkoxy en $C_1$ à $C_6$ et le groupe OM dans lequel M est le sodium ou le potassium;
Y est le groupe $NR^6$ dans lequel $R^6$ est un radical méthyle; et
k est égal à 0.

6. Composé qui est le 2-(4-[(7-chlorocinnoline-3-yl)oxy]phénoxy)propionate d'éthyle ou le 2-(4-[N-(7-chlorocinnoline-3-yl)-N-méthylamino]phénoxy)propionate d'éthyle.

7. Composé de formule VIII

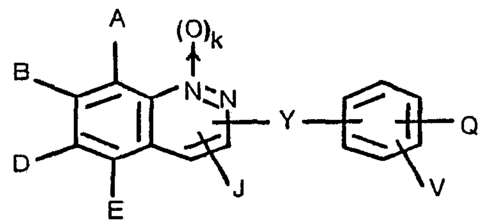

VIII

dans lequel A, B, D, E, J, V, Y et k ont les définitions données dans l'une quelconque des revendications 1 à 5 et Q est choisi entre un groupe hydroxy et un groupe alkoxy en $C_1$ à $C_6$.

8. Composition herbicide, comprenant comme ingrédient actif un composé suivant l'une quelconque des revendications 1 à 6 et un support approprié.

9. Procédé pour endommager gravement ou détruire des plantes non désirées, procédé qui consiste à appliquer auxdites plantes ou au milieu de croissance desdites plantes, une quantité efficace d'un composé suivant l'une quelconque des revendications 1 à 6 ou d'une composition suivant la revendication 8.

10. Procédé pour combattre sélectivement la croissance de mauvaises herbes monocotylédones dans des cultures de dicotylédones, procédé qui consiste à appliquer à ladite culture ou à son milieu de croissance un composé suivant l'une quelconque des revendications 1 à 6 ou une composition suivant la revendication 8 en une quantité suffisante pour endommager gravement ou détruire les mauvaises herbes mais insuffisante pour endommanger notablement la plante cultivée.

11. Procédé suivant la revendication 9 ou 10, dans lequel le composé est appliqué à un taux compris dans l'intervalle de 0,005 à 20 kg par hectare.

12. Procédé de synthèse d'un composé de formule I suivant l'une quelconque des revendications 1 à 6, procédé qui consiste à faire réagir un dérivé de cinnoline de formule IX avec un composé de formule X:

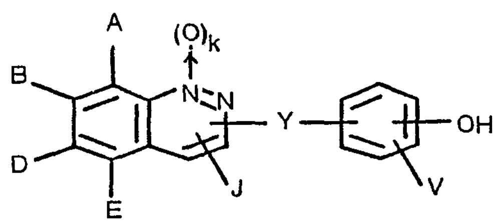

IX

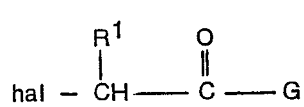

X

dans laquelle A, B, D, E, J, V, Y, k, $R^1$ et G sont tels que définis dans l'une quelconque des revendications 1 à 5 et hal désigne le chlore, le brome ou l'iode, ou à faire réagir un dérivé de cinnoline de formule V avec un composé de formule VI

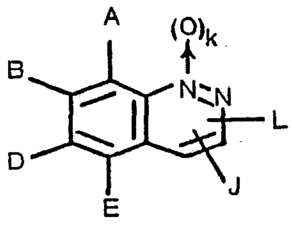

V

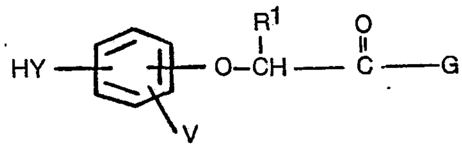

VI

où A, B, D, E, J, V, Y, k, $R^1$ et G sont tels que définis dans l'une quelconque des revendications 1 à 5 et L est un groupe partant.

13. Procédé suivant la revendication 12, dans lequel le composé de cinnoline de formule IX est préparé par réaction d'un dérivé de cinnoline de formule V tel que défini dans la revendication 12 avec un composé de formule VII:

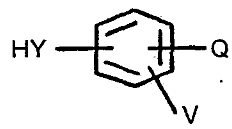

VII

dans laquelle Y et V sont tels que définis dans l'une quelconque des revendications 1 à 5 et Q est un groupe hydroxy ou un groupe alkoxy en $C_1$ à $C_6$, pour former un composé de formule VIII,

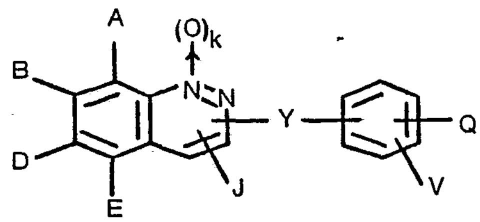

VIII

dans laquelle A, B, D, E, J, V, Y et k sont tels que définis dans l'une quelconque des revendications 1 à 5 et Q est tel que défini ci-dessus et, lorsque Q est un groupe alkoxy en $C_1$ à $C_6$, déalkylation du composé de formule VIII pour former le composé de formule IX tel que défini dans la revendication 12.